(19) Europäisches Patentamt
European Patent Office
Office européen des brevets

(11) Publication number: **0 038 628**
**B1**

# (12) EUROPEAN PATENT SPECIFICATION

(45) Date of publication of patent specification: **10.07.85**

(21) Application number: **81301212.7**

(22) Date of filing: **20.03.81**

(51) Int. Cl.$^4$: **C 08 L 5/08** // (C08L5/08, 89:06)

## (54) Composite material of de-N-acetylated chitin and fibrous collagen, its production and use.

(30) Priority: **21.03.80 JP 36711/80**
**21.03.80 JP 36712/80**

(43) Date of publication of application:
**28.10.81 Bulletin 81/43**

(45) Publication of the grant of the patent:
**10.07.85 Bulletin 85/28**

(84) Designated Contracting States:
**DE FR GB NL SE**

(56) References cited:
**EP-A-0 020 183**
**DE-A-1 492 711**
**GB-A-2 052 518**

**PATENT ABSTRACTS OF JAPAN, vol. 3, no. 55, May 11, 1979, page 141 C 45**
**CHEMICAL ABSTRACTS, vol. 76, no. 17, April 24, 1972, page 344, abstract no. 98205n COLUMBUS, Ohio (US)**

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Kosugi, Junichi**
**No. 22 Daiichi-Kurehasoh, 3-10-13 Nerima**
**Nerima-ku**
**Tokyo (JP)**
Inventor: **Kato, Tadaaki**
**K-407, 3-27 Nakadai Itabashi-ku**
**Tokyo (JP)**
Inventor: **Funabashi, Masayuki**
**56-25 Tsurumaki Takakura-Cho**
**Iwaki-shi Fukushima-ken (JP)**

(74) Representative: **Myerscough, Philip Boyd et al**
**J.A.Kemp & Co. 14, South Square Gray's Inn**
**London, WC1R 5EU (GB)**

**EP 0 038 628 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a composite material comprising de-N-acetylated chitin and fibrous collagen, to its preparation and to its use.

Representative collagen proteins are the scleroproteins contained in connective tissues, bones, teeth, ligaments, tendons, cutises, fasciae, etc. of mammals, birds, etc. They are widely employed as edible casing material, surgical sutures, pieces for vasculoplastic transplantation or artificial skins, etc. Although collagen is superior to other materials used in such fields, it is not always satisfactory.

For instance, as a casing material for foods such as ham and sausage which necessitate smoke-drying, the gut of cattle, swine, sheep, etc., i.e. natural collagen material, has been utilized. However, there are limits to the production of shaped material from natural collagen material. Since this shaped material is produced via various complicated and troublesome treatment steps, it is highly expensive. Besides, since the shape and quality of natural collagen material are irregular, such material has scarcely been used in conjunction with conventional high-speed meat-stuffing machines in the production of ham and sausage.

Tubes made of collagen fibers, i.e. an artificial collagen casing, have been proposed to resolve the above-mentioned demerits. However, artificial collagen fibers are poor film-forming materials. Besides, artificial collagen casings need to possess a large thickness to retain their mechanical strength. Accordingly, they do not possess a good appearance and have a noticeably different fuel when eaten. Further, in hams and sausages encased in such artificial collagen casings, unfavorable phenomena such as breaking during cooking and of ablation of the casing from the stuffed meat take place. Recently, the idea of crosslinking the artificial collagen in a casing has been proposed for resolving the problem during cooking. However, such crosslinked collagen casings do not have a satisfactory feeling in the mouth, either.

Surgical materials such as the surgical sutures, artificial skin and hemostatic material, etc. made from collagen materials are still unsatisfactory from the viewpoint of their biostability (the property of not being absorbed by living body) other than the problem of strength. Providing biostability to a collagen material has been studied from various aspects. The collagen can be crosslinked. However, the shaped material made from crosslinked collagen is noticeably poorer in elasticity than material made from conventional shaped collagen material. In addition, the following process is under trial with the intention of resolving the above-mentioned problems by forming a polyion complex between collagen and a high polymeric substance having carboxyl group(s) or sulfate group(s):

$$H_3N^{\oplus}-\psi-COO^{\ominus}+Y-COO^{\ominus} \text{ or } Y-SO^{\ominus}_3$$

$$\rightarrow Y-COO^{\ominus}H_3N^{\oplus}-\psi-COO^{\ominus} \text{ or}$$

$$Y-SO_3^{\ominus}H_3N^{\oplus}-\psi-COO^{\ominus}$$

wherein $H_3N^{\oplus}-\psi-COO^{\ominus}$ represents a collagen molecule ion and $Y-COO^{\ominus}$ or $Y-SO_3^{\ominus}$ represents an ion or a high polymeric substance. The thus obtained polyion complex is excellent in biostability and has an antithrombogenic property. However, it dissolves in aqueous media, for example in an aqueous solution of an inorganic salt such as sodium chloride, and possesses poor mechanical properties. Accordingly, it is necessary to subject the polyion complex to a further treatment to stabilise it, such as to crosslinking, etc.

It has now been found that the problems of prior art can be overcome by using a composite material incorporating de-N-acetylated chitin and fibrous collagen. Accordingly, the present invention provides a composite material comprising de-N-acetylated chitin and fibrous collagen in a weight ratio of de-N-acetylated chitin to fibrous collagen in the range 0.01:99.99 to 99:1. The material may further comprise up to 40 parts by weight of gelatin and/or soluble collagen per 100 parts by weight of the composite material. The material of the invention may therefore be a two component material of de-N-acetylated chitin and fibrous collagen, a three component material of de-N-acetylated chitin, fibrous collagen and gelatin or soluble collagen, or a four component material of de-N-acetylated chitin, fibrous collagen, gelatin and soluble collagen.

The two component composite comprising de-N-acetylated chitin and fibrous collagen contains 0.01 to 99 parts by weight of the de-N-acetylated chitin to 1 to 99.99 parts by weight of fibrous collagen per 100 parts by weight of the composite material. The three or four component composite can be obtained by partially replacing the fibrous collagen of the two component composite material with gelatin and/or soluble collagen, wherein the amount of gelatin and/or soluble collagen is up to 40 parts by weight per 100 parts by weight of the composite material.

The composite material is prepared according to the invention by bringing de-N-acetylated chitin into contact with fibrous collagen and optionally gelatin and/or soluble collagen in an acidic medium and deacidifying the product thus obtained, so as to obtain the composite material. The pH of the aqueous acidic medium can be from 1 to 6, preferably from 3 to 6. The deacidified product can be crosslinked, if necessary.

Proteinic composite materials such as those comprising collagen fiber and a soluble protein or collagen fiber and gelatin, have been reported. However, since in such proteinic compositions the bonding between collagen and the soluble component is weak, there is a noticeable reduction in the strength of the

composition when it is brought into hot water. On the other hand, composite materials according to the present invention do not dissolve in aqueous salt solutions and can possess excellent mechanical strength which is not particularly reduced in hot water. It is possible to obtain materials which are not absorbed by living bodies, coagulate blood, resist attack by bacteria, posssess film-forming ability and adhere closely to stuffed materials such as meat.

Although it has not yet been elucidated why the composite material according to the present invention can possess such properties, the existence of the strong bonding between the de-N-acetylated chitin and the proteinaceous fibrous collagen, gelatin and soluble collagen is presumed to form a stable polyion complex as follows:

$$H_3N^{\oplus}-\psi-COO^{\ominus}+Z-N^{\oplus}H_3 \rightarrow H_3N^{\oplus}-\psi-COO^{\ominus}H_3N^{\oplus}-Z$$

wherein $H_3N^{\oplus}-\psi-COO^{\ominus}$ represents an ion of a proteinaceous molecule and $H_3N^{\oplus}-Z$ represents an ion of the de-N-acetylated chitin molecule.

The composite material according to the present invention is applicable to various usages. One such usage is as an edible casing material. Besides, as Kiriyama et al have reported in A.N.I. No. 71, since chitosan has the following physiological activities:

a) returning to normal raised levels of serum cholesterol,
b) inhibiting the raising of the level of blood sugar,
c) removing growth-inhibiting substances and
d) preventing cancer of the colon,

composite materials according to the present invention may be added to foods in the form of spherically shaped or fibrous material, or may exhibit preventive or therapeutic effects to the various symptoms such as a) to d) as an edible material.

Furthermore, since the composite material according to the present invention is not absorbable by living bodies because of the presence of de-N-acetylated chitin, and also has a blood-coagulating property, it can be surgically or therapeutically applied in the forms of films, fibers and materials having a three dimensional structure. Although the crosslinked or uncrosslinked composite materials are themselves thrombogenic, these can be applied as an anti-thrombogenic material after being treated with heparin. It is possible to use the materials medically for artificial vessels, artificial skins, artificial kidneys, etc.

In addition, since the composite material according to the present invention is excellent in adsorbing proteins, it is possible to use it as a base material for immobilizing enzymes and microorganisms or as an adsorbent of enzymes and microorganisms.

The de-N-acetylated chitin preferably has, from the viewpoint of solubility and processability, a degree of de-N-acetylation of 50 to 100%, preferably 70 to 100% and a viscosity of $2\times10^{-2}$ to 1 Pa s (20 to 1000 cp), the viscosity being determined at 20°C on an aqueous 1% by weight solution of acetic acid containing 0.5% by weight of the de-N-acetylated chitin. Commercialized chitosan or the product obtained by heating chitin in an aqueous alkali solution of high concentration may be used. The chitin can be obtained from shells of arthropods such as crabs, lobsters, shrimps, etc. by conventional separation and purification techniques.

The protein used in the present invention is fibrous collagen, which is insoluble in an aqueous solvent, and optionally gelatin and soluble collagen, which are soluble in an aqueous solvent. These proteins are hereinafter referred to as the proteinic component. The proteinic component is obtainable by separating and purifying proteinaceous tissues from living bodies of various animals, or by chemically treating the separated and purified protein. The aqueous solvent is an aqueous solution of acid or alkali. The acid may be an inorganic acid such as hydrochloric acid, a single organic acid such as acetic acid, propionic acid, adipic acid, or a mixture of two or more kinds of organic acid. The alkali is a conventional alkali such as sodium hydroxide, potassium hydroxide, etc.

Collagen fiber can be obtained by chemically or mechanically removing non-collagenic components from the fibrous components constituting major protein which is present in the connecting tissues of animals. Collagen fiber, for example, may be prepared by a process wherein bovine hide or bovine Achilles tendon, after having been subjected to a depilating treatment, is finely cut by a mincing machine and is swollen in an aqueous acidic or alkaline medium, and then is disintegrated by a grinder to obtain an aqueous dispersion, followed by filtration, if necessary.

In order to improve the strength and homogeneity of the composite material that is produced it is preferable to subject the collagen, for example the finely cut hide or tendon, to a pretreatment. The pretreatment may be crosslinking of the amino group of lysine residues in collagen by a crosslinking agent such as formaldehyde, glutaraldehyde, dialdehyde starch, glyoxal, smoke-drying liquid, epihalohydrin, etc., succinylation of the amino groups by succinic anhydride, acylation of the amino groups by carboxylic acid anhydride or esterification of carboxyl groups in the side chain of aspartic acid residues or glutamic acid residues in collagen.

As the raw material for the collagen fibers, scrap collagen remaining after the preparation of natural or artificial collagen casings which are not utilizable for such casings can be employed.

The fibrous collagen is preferably $10^{-6}$ to $3\times10^{-6}$ m (1 to 3 μ) in diameter and $10^{-4}$ to $1.5\times10^{-2}$ m (0.1 to 15 mm) in length, and from 5,000 to 1,000,000, preferably 20,000 to 500,000 in molecular weight, although not necessarily being restricted to such ranges.

The gelatin may be of the type used in the food industry. The soluble collagen, soluble in an aqueous solvent, may be obtained by treating the fibrous collagen with proteinase, an acid or an alkali to become wholly soluble in the aqueous solvent. A partly solubilized product obtainable by treatment of fibrous collagen is usable as a mixture of the fibrous collagen and the soluble collagen.

The soluble gelatin and soluble collagen are employed in an amount of 0 to 40 parts by weight per 100 parts by weight of the composite material. When the amount of gelatin, soluble collagen or mixture thereof is more than 40 parts by weight, the moisture content of the composite material that is obtained is so much that it is difficult to maintain the shape of the composite material and its tensile strength is poor. The use of gelatin is preferable as the soluble protein. The amount of the gelatin is preferably 5 to 30 parts by weight per 100 parts by weight of the composite material. The soluble protein, as a component of the composite material, contributes to the pliability, reduced thermal deformation temperature, improved mechanical strength, and also improved dietary feeling of the composite material.

As indicated above, the composite material is prepared according to the invention by first bringing the de-N-acetylated chitin and the proteinic component into contact in an acidic medium, for example having a pH value in the range of 1 to 6, preferably of 3 to 6, on deacidifying of the product thus obtained, followed by crosslinking if necessary.

The deacidifying treatment mentioned herein means adjusting the pH of the reaction product of the de-N-acetylated chitin and the proteinic component to a value which is higher than the isoelectric point of the reaction product. The deacidificating treatment is accomplished, for instance, by addition of an alkaline solution such as sodium hydroxide, potassium hydroxide or ammonium hydroxide to bring the pH to above 7, by addition of an aqueous solution of alkaline salt, by removal of acid present in the reaction mixture by evaporation or by treatment by electrodeposition.

The gelatin obtained by denaturing collagen can have various isoelectric points, jelly strengths, ash contents, etc. depending on the denaturation treatment. Accordingly, where a proteinaceous composite material is prepared by electrodeposition of the gelatin obtained by an acid-treatment, it is necessary to adjust the pH of the electrodeposition-controlling liquid to higher than 7 to deposit the composite material on the cathode. On the other hand, in the case where gelatin obtained by alkalitreatment is used, the composite material must be formed on the anode by adjusting the pH of the electrodeposition-controlling liquid to lower than 7. On the other hand, in the present invention, the composite material can also be prepared by electrodeposition without the above-mentioned restriction owing to the presence of the de-N-acetylated chitin. Meanwhile, as the gelatin for use in the case of shaping by electrodeposition, a product containing less than 0.5% by weight of ash, preferably less than 0.2% by weight of ash, is desirable from the viewpoints of quality and electricity consumption in preparation.

According to the present invention, by combining the proteinic component which has insufficient shape-forming property with the de-N-acetylated chitin, the shape-forming property, for example membrane-forming property, can be improved and problems concerning the preparation of the composite material such as yield, stability and uniformity of the product can be overcome. Besides, since the residual free aldehyde remaining after the crosslinking pretreatment of the fibrous collagen is also reacted with the de-N-acetylated chitin, the composite material is desirable from the viewpoint of safety.

The preparation of the composite material of the present invention will now be described in more detail.

As one way of contacting the proteinic component and the de-N-acetylated chitin, an acid medium containing the de-N-acetylated chitin and a medium containing a proteinic component can be mixed and the mixture dispersed. In this case, the concentration of the de-N-acetylated chitin in the acidic medium is preferably less than 5% by weight, more preferably less than 1% by weight, because of its viscosity (the medium being an aqueous dilute solution of acetic acid, hydrochloric acid, etc.), and the pH of the acidic medium is preferably 1 to 6, preferably 3 to 6. It is not always necessary for the de-N-acetylated chitin to be homogeneously dissolved in the medium. The de-N-acetylated chitin may be dispersed uniformly as a fibrous state. The medium containing the proteinic component may be prepared by dispersing the proteinic component in an aqueous acidic solution of hydrochloric acid, etc. and by adjusting the pH thereof to 3 to 6, preferably 3 to 4. However, the medium need not necessarily be acidic. It may be neutral or alkaline and in this case, it is necessary to ensure that the pH of the mixture of the medium containing the de-N-acetylated chitin and the medium containing the proteinic component is in the acidic region. In addition, in the case of electrodeposition, the medium containing the proteinic component is used in an acidic state.

In the present invention, a film-like composite material can be prepared by pouring a de-bubbled mixture of the medium containing the de-N-acetylated chitin and the medium containing the proteinic component onto a glass plate and then de-acidifying the poured mixture by the use of a hot air drier. The film-like composite material is characterised in that its strength does not depend on the tensile direction, that is longitudinal or transverse, in spite of having fibrous protein. This characteristic feature of film-like composite materials according to the present invention can be exhibited by the presence of the de-N-acetylated chitin.

Spherically shaped composite material can be prepared by dropping a de-bubbled mixture of the two media into a hydrophobic organic solvent such as toluene or xylene and then treating the thus formed material with an aqueous alcoholic solution to deacidify the spherically shaped material. It is possible to

prepare fibers, films or hollow composite materials by extruding the mixture from a hole-like or slit-like nozzle(s) into a highly concentrated aqueous solution of sodium chloride or ammonium hydroxide. In addition, the composite material can be obtained by electrodeposition in which an aqueous medium containing the de-N-acetylated chitin and the proteinic component is introduced into an electrolytic cell provided with at least one cathode and at least one anode, and a direct voltage is applied between the electrodes to make the composite material accumulate on the surface of a predetermined electrode.

Tube-like composite materials according to the present invention have an extremely thin wall membrane excellent in strength, and accordingly, are highly suitable for edible casing materials with a desirable dietary feeling.

Where composite materials according to the present invention are used for edible casings, it is preferable that the amount of de-N-acetylated chitin be from 0.01 to 60 parts by weight, of fibrous collagen from 40 to 99.99 parts by weight and, if employed, of gelatin, soluble collagen or mixture thereof from 5 to 30 parts by weight per 100 parts by weight of the composite material from the viewpoints of the dietary feeling, the strength for smoke-drying and cooking, thermal deformation temperature and heat-stability. On the other hand, in the case where composite materials according to the present invention are used as food additives, the amount of de-N-acetylated chitin is preferably from 1 to 99 parts by weight to 100 parts by weight of the composite material and it is preferable to increase the amount of the de-N-acetylated chitin.

As another way of contacting the proteinic component and de-N-acetylated chitin, proteinic materials such as sheets, films, tubes, fiber-like forms, etc. are immersed in an acidified medium containing the de-N-acetylated chitin and then the material is subjected to deacidification. In this case, since the exterior of the composite material is composed of the de-N-acetylated chitin component, such a shaped composite material can show unique properties such as improved mechanical properties, improved thermal resistance and anti-bacterial property.

Furthermore, where the composite material according to the present invention obtained by one of the abovementioned methods is further subjected to a crosslinking treatment, the crosslinked composite material exhibits improved blood-coagulation properties and improved resistance to acids.

For the crosslinking treatment, a composite material according to the present invention can be immersed in an aqueous solution prepared by dissolving a crosslinking agent in a buffer solution of phosphoric acid at a pH of 7.4 for 0.1 to 1 hour at 10 to 50°C. After the crosslinking is over and the crosslinked material is washed with water, a water-insoluble crosslinked composite material is obtained.

Since it is possible by such a crosslinking process to cause not only crosslinking of the de-N-acetylated chitin but also crosslinking between the de-N-acetylated chitin and the proteinic component, an improved bonding force in the surface of the composite material is obtained. The crosslinking occurs between the same or different functional groups among amino groups and hydroxyl groups in the de-N-acetylated chitin and collagen. As a crosslinking agent, the agents mentioned previously in connection with the crosslinking pretreatment of the fibrous collagen can be employed.

The following Examples illustrate the invention.

Example 1

After immersing 100 kg of a salted steer hide in water for 15 hours, washing it with water, and defatting it, 90 kg of the thus treated hide was immersed into 450 kg of an aqueous liquid containing 2% by weight of calcium hydroxide, 0.5% by weight of sodium sulfide and 0.5% by weight of diethylamine at 25°C for 24 hours while gently stirring the mixture. The thus treated hide was subjected successively to a depilating roll to remove hairs and decomposition products formed by the treatment with calcium hydroxide, to a splitting machine to roughly divide the hide, to a meat slicer to cut the divided hide into tapes of $5\times10^{-3}$ m (5 mm) in width and then to a mincing machine to cut the tapes into $5\times10^{-2}$ m (5 cm) lengths.

Eighty kilograms of the thus obtained hide (referred to as the refined hide hereinafter) were dispersed into 800 kg of water, and 3 kg of acetic acid were added to the dispersion. After stirring gently for 12 hours, the refined hide was separated from the liquid, dehydrated by centrifugation and washed with de-ionized water until the electric conductivity of the washings became less than $2\times10^{-3}$ mho/m (20 μ mho/cm). At this time, the ash content of the refined hide was less than 0.1%. Forty kilograms of the thus treated refined hide were dispersed in 400 kg of an aquoeus solution containing 0.015% by weight of glutaraldehyde and adjusted to pH of 3.0 by the addition of hydrochloric acid, and then crosslinked while stirring the solution gently for 12 hours at 20°C.

The crosslinked refined hide was collected by filtration with a wire netting and subjected to a pulp-refiner together with 113 kg of cold water to beat the fiber-bundles of the refined hide resulting in a dispersion of collagen fibers, to which hydrochloric acid was further added to make the pH thereof 3.0. The thus obtained dispersion weighing 125 kg contained 2.5% by weight of crosslinked collagen comprising a mixture of collagen fibers of $10^{-6}$ to $3\times10^{-6}$ m (1 to 3 μ) in diameter and of $10^{-3}$ to $10^{-2}$ m (1 to 10 mm) in length and still finer collagen fibrils.

Meanwhile, into 10 kg of an aqueous hydrochloric acid solution of pH of 3, one kg of de-N-acetylated chitin having a degree of de-N-acetylation of 95% was dissolved. After adjusting the pH of the solution to 3.0, 20 kg of an aqueous solution of de-N-acetylated chitin were obtained.

Into 1.5 kg of the aqueous dispersion of crosslinked collagen fiber, 50 kg of de-ionized water were

added. The mixture was stirred at a high speed. Then $3.74\times10^{-1}$ kg (374 g) of the aqueous solution of de-N-acetylated chitin was added to the dispersion. This mixture was stirred for one hour to form an aqueous mixture containing collagen fiber and de-N-acetylated chitin at a weight ratio of 100/5, the viscosity and the electric conductivity of the aqueous mixture being $2\times10^{-1}$ Pa s (200 cP) and $2.5\times10^{-2}$ mho/m (250 μv/cm), respectively.

From the aqueous mixture of collagen fiber and de-N-acetylated chitin, a tubular membrane was formed by electrodeposition in which a membrane was formed on the cathode (17.5 mmφ) by electrophoresis of collagen fiber and de-N-acetylated chitin. The thus formed membrane was pulled up the velocity of pulling up being 10 m/min ($1.66\times10^{-1}$ m/s).

The thus prepared tubular membrane just after electrodeposition contained 2 kg of water per kg of dry membrane and showed a tensile strength of $2\times10^{6}$ kg/m$^2$ (200 kg/cm$^2$). The membrane, after immersion into an aqueous 7% by weight glycerol solution, was dried by hot air at 75°C for 2 min, while holding the tubular membrane by pressurized air of $2.94\times10^{3}$ Pa (300 mm $H_2O$) to be the composite material (Specimen A) according to the present invention.

For comparison, a tubular membrane (Comparative Specimen A) consisting only of collagen fiber was prepared by a similar process to that mentioned above except for not adding the de-N-acetylated chitin to the crosslinked collagen fiber. The Comparative Specimen A contained 7 kg of water per kg of the dry membrane just after electrodeposition showing a tensile strength of $10^{6}$ kg/m$^2$ (100 kg/cm$^2$).

The results of determination of tensile strength and tear strength in the wet state of Specimen A and Comparative Specimen A according to the method described in JIS P-8113 and JIS P-8116 are shown in Table 1. As will be seen in Table 1, the specimen prepared from a mixture of crosslinked collagen fiber and de-N-acetylated chitin showed a noticeably improved tensile strength, and it was possible to obtain an extremely thin membrane.

Table 2 shows the results of a strength-determination of the membranes by the same method as mentioned above after immersion in an aqueous 0.2 N sodium chloride solution at 20°C for 24 hours. As will be seen in Table 2, the composite material according to the present invention is superior to the Comparative Specimen A in strength, which suggests not only the ionic bonding between collagen fiber and de-N-acetylated chitin but also another bonding factor.

TABLE 1

| Specimen | Thickness of membrane $10^{-6}$ m(μ) | Tensile strength Kg/m$^2$ (kg/cm$^2$) | Tear strength Kg m/m (g cm/cm) | Elongation at break (%) |
|---|---|---|---|---|
| Specimen A | 10 | $4.6\times10^{6}$ (460) | $3.6\times10^{-2}$ (36) | 32 |
| Comparative Specimen A | 15 | $2.5\times10^{6}$ (250) | $2.5\times10^{-2}$ (25) | 25 |

TABLE 2

| Specimen | Tensile strength Kg/m$^2$ (kg/cm$^2$) | Tear strength Kg m/m (g cm/cm) | Elongation at break (%) |
|---|---|---|---|
| Specimen A | $2.75\times10^{6}$ (275) | $6.1\times10^{-2}$ (61) | 115 |
| Comparative Specimen A | $1.30\times10^{6}$ (130) | $4.8\times10^{-2}$ (48) | 64 |

Example 2

One kg of an acid-treated gelatin (lot number of F-795, manufactured by Miyagi Chem. Co. Ltd., Japan) was dissolved in 10 kg of an aqueous hydrochloric acid solution of pH 3. After adjusting the pH to 3.0, 20 kg of an aqueous solution of gelatin was obtained.

After adding 50 kg of de-ionized water to 15 kg of the aqueous dispersion of crosslinked collagen fiber prepared in Example 1 and stirring rapidly, $3.74\times10^{-1}$ Kg (374 g) of the aqueous solution of de-N-acetylated chitin prepared in Example 1 and $3.74\times10^{-1}$ Kg (374 g) of the aqueous solution of gelatin were further added to the dispersion of crosslinked collagen fiber. The whole mixture was stirred for one hour to obtain

an aqueous mixture of collagen fiber, de-N-acetylated chitin and gelatin at a weight ratio of collagen: de-N-acetylated chitin: gelatin of 100:5:5. From the thus obtained aqueous mixture, a tubular membrane was formed by the same method as in Example 1 (Specimen B).

For comparison, a tubular membrane (Comparative Specimen B) consisting of collagen and gelatin was prepared by the same process, however without adding de-N-acetylated chitin.

The results of determination of tensile strength and tear strength of the products by the same methods as described in Example 1 in wet state are shown in Table 3. As is seen in Table 3, the strength of the membrane containing collagen fiber, de-N-acetylated chitin and gelatin was improved as compared to that of the membrane only containing collagen fiber and gelatin, thus showing the effect of added de-N-acetylated chitin. Furthermore, the results of determination of the mechanical properties of the products after immersion in warm water at 40°C for 24 hours shown in Table 4 indicate the superiority of the composite material according to the present invention in strength compared with the product obtained without adding de-N-acetylated chitin.

TABLE 3

| Specimen | Thickness $10^{-6}$ (μ) | Tensile strength Kg/m$^2$ (kg/cm$^2$) | Tear strength kg m/m (g cm/cm) | Elongation at break (%) |
|---|---|---|---|---|
| Specimen B | 10 | $4.8\times10^6$ (480) | $3.6\times10^{-2}$ (36) | 35 |
| Comparative Specimen B | 10 | $3.4\times10^6$ (340) | $2.7\times10^{-2}$ (27) | 27 |

TABLE 4

| Specimen | Tensile strength Kg/m$^2$ (kg/cm$^2$) | Tear strength Kg m/m (g cm/cm) | Elongation at break (%) |
|---|---|---|---|
| Specimen B | $3.7\times10^6$ (370) | $6\times10^{-2}$ (60) | 110 |
| Comparative Specimen B | $1.4\times10^6$ (140) | $4.5\times10^{-2}$ (45) | 60 |

Example 3

A tubular composite material (Specimen C) was prepared in the same manner as in Example 1 except for the weight ratio of the collagen fiber to de-N-acetylated chitin of 100/20 and a membrane-pulling-up velocity of $4.17\times10^{-1}$ mls (25 m/min). For comparison, a single material (a dispersion of collagen fiber of concentration of 0.58% by weight) was processed to form a membrane; however this membrane could not be continuously pulled up.

The mechanical properties of Specimen C were: $8\times10^{-6}$ m (8 μ) in thickness, $5.1\times10^6$ kg/m$^2$ (510 kg/cm$^2$) in tensile strength, 32% in elongation at break and $3.2\times10^{-2}$ kg m/m (32 g cm/cm) in tear strength.

Example 4

A tubular composite material (Specimen D) was prepared in the same manner as in Example 1 and 2 except for the weight ratio of collagen fiber: de-N-acetylated chitin: gelatin of 100:20:5 and a membrane-pulling-up velocity of $4.17\times10^{-1}$ m/s (25 m/min). For comparison, it was attempted to form a membrane from an aqueous mixture of dispersed collagen fiber and gelatin at a weight ratio of collagen fiber: gelatin of 100:5; however, this membrane could not be continuously pulled up. The mechanical properties of Specimen D were: $8\times10^{-6}$ m (8 μ) in thickness, $4.9\times10^6$ Kg/m$^2$ (490 kg/cm$^2$) in tensile strength, 33% in elongation at break and $3.3\times10^{-2}$ kg m/m (33 g cm/cm) in tear strength.

Example 5

A tubular composite material (Specimen E) was prepared in the same manner as in Example 1 and 2 except for using alkali-treated gelatin (lot number of F-800, Miyagi Chem. Co. Ltd., Japan) instead of acid-treated gelatin of Example 2, a weight ratio of collagen fiber: gelatin: de-N-acetylated chitin of 100:20:10 and a membrane-pulling-up velocity of $2.5\times10^{-1}$ m/s (15 m/min.). For comparison, it was

attempted to deposit a membrane from an aqueous mixture of collagen fiber and gelatin at a weight ratio of collagen to gelatin of 100:20; however no membrane was deposited on the electrode. The mechanical properties of Specimen E were: $9\times10^{-6}$ m (9 μ) in thickness, $4.4\times10^{6}$ kg/$m^2$ (440 kg/$cm^2$) in tensile strength, 34% in elongation at break and $3\times10^{-2}$ m/m (30 g cm/cm) in tear strength.

Example 6

After mixing 10 kg of the dispersion of crosslinked collagen fiber obtained in Example 1 and 1.5 kg of an aqueous 5% by weight of de-N-acetylated chitin solution obtained in Example 1 while stirring, the mixture was de-bubbled under a reduced pressure. The de-bubbled mixture was extruded from a slit of $10^{-1}$ m (10 cm) in width and of $3\times10^{-4}$ m (0.3 mm) in height into an aqueous 0.1 N ammonium hydroxide solution contained in a coagulation bath. The thus obtained film was washed with water, and was treated in the same manner as in Example 1 to be a film composite material according to the present invention (Specimen F).

The properties of the film determined in the same manner as in Example 1, were: $3\times10^{-5}$ m (30 μ) in thickness, $5.2\times10^{6}$ kg/$m^2$ (520 kg/$cm^2$) in tensile strength, 30% in elongation at break and $7.5\times10^{-2}$ kg m/m (75 g cm/cm) in tear strength.

On the other hand, a film prepared from the same dispersion of crosslinked collagen fiber in the same manner as above (Comparative Specimen C) without adding the solution of de-N-acetylated chitin showed the following properties: $3\times10^{-5}$ m (30 μ) in thickness, $2\times10^{6}$ kg/$m^2$ (200 kg/$cm^2$) in tensile strength, 26% in elongation at break and $4.8\times10^{-2}$ kg m/m (48 g cm/cm) in tear strength.

From the above-mentioned results, the composite material according to the present invention was confirmed as superior to the single material (only consisting of crosslinked collagen fiber) in mechanical strength.

Example 7

After mixing 10 kg of the dispersion of crosslinked collagen fiber obtained in Example 1, 1 kg of an aqueous 5% by weight gelatin solution obtained in Example 2 and 1.5 kg of an aqueous 5% by weight of de-N-acetylated chitin solution obtained in Example 1, the mixture was stirred well and de-bubbled under a reduced pressure, and subjected to the same procedures as in Example 6 to obtain a film composite material according to the present invention (Specimen G). The mechanical properties of Specimen G in wet state were: $3\times10^{-5}$ m (30 μ) in thickness, $4.95\times10^{6}$ kg/$m^2$ (495 kg/$cm^2$) in tensile strength, 31% in elongation at break and $7.3\times10^{-2}$ kg m/m (73 g cm/cm) in tear strength. On the other hand, a membrane prepared from a aqueous mixture of collagen fiber and gelatin in the same manner as this Example (Comparative Specimen D) showed mechanical properties of: $3\times10^{-5}$ m (30 μ) in thickness, $3.2\times10^{6}$ kg/$m^2$ (320 kg/$cm^2$) in tensile strength, 28% in elongation at break and $6.5\times10^{-2}$ kg m/m (65 g cm/cm) in tear strength. This showed that the composite material according to the present invention can possess excellent strength.

Example 8

Aptitude test in mechanical stuffing

Practical tests were carried out on the tubular Specimen C obtained in Example 3 and the tubular Comparative Specimen A obtained in Example 1 by using them as sausage-casing for a sausage meat of the following composition:

| | |
|---|---|
| salted pork meat | 1.3 kg |
| lard meat | $7.5\times10^{-1}$ kg |
| albumen | $9\times10^{-2}$ kg |
| iced water | $7.5\times10^{-1}$ kg |
| starch | $1.2\times10^{-1}$ kg |
| sodium glutamate | $9\times10^{-3}$ kg |
| allspice | $1.2\times10^{-2}$ kg |
| sucrose | $9\times10^{-3}$ kg |
| a phosphate | $6\times10^{-3}$ kg |
| pepper powder | $6\times10^{-3}$ kg |

Table 5 shows the results when the sausage meat was stuffed into the two kinds of sausage-casings (Specimen C and Comparative Specimen A) using a semi-automatic stuff-worker 760.

As is seen in Table 5, the casings prepared from a mixture of crosslinked collagen fiber and de-N-acetylated chitin (Specimen C) were able to withstand the severe conditions of stuffing. In addition, the knot on the sausage prepared from the sausage-casing according to the present invention (Specimen C) was smaller than that of from Comparative Specimen A and superior in appearance.

TABLE 5

| Sample* | Addition of de-N-acetylated chitin | Number of runs | Stuffed amount (kg/piece) | Length of Sausage after stuffing $m \times 10^{-3}$ (cm) | Rate of rupture |
|---|---|---|---|---|---|
| 1 | yes | 3 | $1.8 \times 10^{-2}$ | 9.5 | 0 |
| 2 | yes | 9 | $2.0 \times 10^{-2}$ | 9.8 | 0 |
| 3 | yes | 1 | $1.45 \times 10^{-2}$ | 7.3 | 0 |
| 4 | yes | 1 | $1.75 \times 10^{-2}$ | 8.4 | 0 |
| 5 | yes | 1 | $2.0 \times 10^{-2}$ | 9.9 | 0 |
| 6 | yes | 1 | $2.35 \times 5.10^{-2}$ | 11.8 | 0 |
| 7 | no | 1 | $1.72 \times 10^{-2}$ | 9.5 | 0 |
| 8 | no | 2 | $1.94 \times 10^{-2}$ | 10.5 | 1/400 |
| 9 | no | 1 | $1.54 \times 10^{-2}$ | 7.4 | 1/250 |

Notes:

(*) Sample No. 1 to 6 used Specimen C according to the present invention. Sample No. 7 to 9 used Comparative Specimen A.

(**) In one run 1000 m of the tubular material is produced by the electrodeposition procedure.

Example 9

Aptitude test in mechanical stuffing

The same practical tests were carried out on the tubular Specimen D obtained in Example 4 and Comparative Specimen B obtained in Example 2 as sausage-casings with the same sausage meat as in Example 8. Table 6 shows the results of stuffing when the sausage meat was stuffed into the two kinds of sausage-casings (Specimen D and Comparative Specimen B) while using a semi-automatic stuff-worker 760.

As is seen in Table 6, the casings prepared from an aqueous mixture of dispersed collagen and de-N-acetylated chitin and gelatin (Specimen D) according to the present invention were able to withstand the severe conditions of stuffing. In addition, the knot on the sausages prepared from the sausage-casing according to the present invention (Specimen D) was smaller than that of Comparative Specimen B and superior also in appearance.

TABLE 6

| Sample No. | Addition of de-N-acetylated chitin | Number of runs | Stuffed amount (kg/piece) | Length of sausage $m\times10^{-3}$ (cm) | Rate of rupture |
|---|---|---|---|---|---|
| 1 | yes | 3 | $1.8\times10^{-2}$ | 9.5 | 0 |
| 2 | yes | 9 | $2.0\times10^{-2}$ | 9.8 | 0 |
| 3 | yes | 1 | $1.45\times10^{-2}$ | 7.3 | 0 |
| 4 | yes | 1 | $1.75\times10^{-2}$ | 8.4 | 0 |
| 5 | yes | 1 | $2.0\times10^{-2}$ | 9.9 | 0 |
| 6 | yes | 1 | $2.35\times10^{-2}$ | 11.8 | 0 |
| 7 | no | 1 | $1.72\times10^{-2}$ | 9.5 | 0 |
| 8 | no | 2 | $1.94\times10^{-2}$ | 10.5 | 1/1000 |
| 9 | no | 1 | $1.54\times10^{-2}$ | 7.4 | 1/500 |

Example 10

Sausages of Sample No. 2 and No. 8 prepared in Example 8 were smoked as follows:

Pieces of sausage were hung from a bar with eight rings in chains, and put into a smoking chamber. They were dried for 30 min. at a controlled temperature of 55 to 60°C at first, and then the temperature was raised to 75°C while introducing smoke generated from wood chips and further introducing steam into the chamber to control the humidity of the chamber at a constant level, the smoking treatment being continued for 30 min. Then, steam was again introduced into the chamber to raise the temperature to 75 to 80°C and after maintaining that temperature for 50 min., the sausages were taken out of the chamber and cold water was sprayed onto the sausages to quench them. These sausages were subjected to the following rupture tests under conditions simulating their cooking at home by housewives:

1) Fry-test

A frying oil was poured into a frying pan provided with a temperature controller. While keeping the temperature of the oil at 160°C, sausages were put into the pan two at a time until ten sausages had been added (to prevent a reduction in the oil temperature). The number of sausages which ruptured within 30 sec. of being put into the pan were counted.

2) Frying pan test

A frying pan was set so that the bottom of the pan was immersed in an oil bath and was $10^{-2}$ m (10 mm) lower than the surface of the oil. The temperature of the oil bath was controlled so that the surface of the pan was kept at 175 to 180°C. Then, a small amount of frying oil was painted onto the whole surface of the pan and five sausages were put into the pan and moved within the pan slowly for 2 min. The number of ruptured sausages was counted. The test was carried out twice on each Sample.

3) Boiling test

While keeping water boiling in a pot $5\times10^{-2}$ m (50 mm) deep five sausages were added and the number of sausages ruptured within 5 min. of boiling was counted. The test was carried out twice on each Sample.

The results of the three tests are shown in Table 7.

TABLE 7

| Sample No. | Addition of de-N-acetylated chitin | Number ruptured per 10 sausages | | |
|---|---|---|---|---|
| | | Fry-test | Frying pan test | Boiling test |
| 2 | yes | 1 | 0 | 1 |
| 8 | no | 5 | 2 | 10 |

As is seen in Table 7, the sausage-casing of the material according to the present invention showed excellent results in practical cooking tests.

In order to support these practical facts, the respective membranes of these two kinds of sausage casing were immersed into a hot water at 75°C for one min., and their shrinkage (in percent) and the thermal deformation temperature were determined. The results are shown in Table 8.

TABLE 8

| Specimen | Addition of de-N-acetylated chitin | Thermal deformation temperature (°C) | Percent shrinkage | |
|---|---|---|---|---|
| | | | Longitudinal direction | Transversal direction |
| Specimen C | yes | 54.5 | 8.5 | 10.5 |
| Comparative Specimen A | no | 52 | 35.8 | 33.8 |

Also, the sausage with the casing prepared from crosslinked collagen fiber and de-N-acetylated chitin gives a feeling of a mellow taste and sweetness when taken into one's mouth not hitherto felt on other sausages, for instance, with a membrane prepared from collagen fibers only.

Example 11

In the same manner as in Example 1, tubular materials having weight ratios of collagen fiber to de-N-acetylated chitin of 100/1 and 100/0.1, respectively, were prepared. A meat mixture was stuffed into these tubular materials under the conditions of Sample No. 2 of Example 8. The resulting sausages were smoked in the same manner as in Example 10. No rupture of the sausages was observed in stuffing, and the results of the cooking tests carried out as in Example 10 are shown in Table 9.

TABLE 9

| Amount of de-N-acetylated chitin added per 100 parts by weight of collagen fiber | Number ruptured per 10 sausages | | |
|---|---|---|---|
| | Test 1 | Test 2 | Test 3 |
| 1 | 1 | 0 | 1 |
| 0.1 | 2 | 0 | 2 |

Notes:
Test 1: Fry-test,
Test 2: Frying pan test, and
Test 3: Boiling test

As is seen in Table 9, the composite material according to the present invention showed an excellent result even in practical cooking tests.

Example 12

Sausages of Sample No. 2 and No. 8 prepared in Example 9 were smoked in the same manner as in Example 10. The smoked sausages were subjected to the rupture tests under the simulated cooking conditions as in Example 10. The results of the three tests are shown in Table 10.

TABLE 10

| Sample No. | Addition of de-N-acetylated chitin | Number ruptured per 10 sausages | | |
|---|---|---|---|---|
| | | Fry-test | Frying pan-test | Boiling test |
| 2 | yes | 1 | 0 | 3 |
| 8 | no | 3 | 2 | 10 |

As is seen in Table 10, the sausage-casing of the composite material according to the present invention showed excellent results in practical cooking tests. Additionally, when the sausages prepared from the aqueous mixture containing also de-N-acetylated chitin were put into a consumer's mouth, a mellow taste and sweetness spread throughout the mouth giving an unparalleled feeling of eating never given by the sausages using only collagen as the casing material. In sensory tests by a panel consisting of 30 persons, the general evaluation was that no artificial feeling was given by sausages stuffed in casings of the composite material of the present invention as compared to sausages stuffed in natural sheep gut.

Example 13

Into $10^{-1}m^3$ (100 litres) of acetic anhydride at a temperature of 15°C, 50 kg of the refined hide dehydrated by centrifuging in Example 1 were immersed for 8 hours to acylate the collagen fibers in the hide. The thus treated refined hide was washed with flowing de-ionized water. The isoelectric point of the original steer hide of pH 6.5 changed to 3.8 as a result of the acetylation. An aqueous dispersion of collagen fibers was prepared from the acetylated hide in the same manner as in Example 1, the content of collagen being 2.5%.

An aqueous solution of de-N-acetylated chitin obtained in Example 1 was added to the thus prepared aqueous dispersion of collagen fiber in the same manner as in Example 1, however with a ratio of collagen to de-N-acetylated chitin of 100/10, to prepare an aqueous mixture of collagen fiber and de-N-acetylated chitin. An electrodeposited membrane was prepared from the aqueous mixture as in Example 1 with the same after-treatment as in Example 1. The mechanical properties of the thus prepared tubular membrane, determined as in Example 1, were: $9\times10^{-6}$ m (9 μ) in thickness, $4.85\times10^{6}$ kg/m$^2$ (485 kg/cm$^2$) in tensile strength, 33% in elongation at break and $3.5\times10^{-2}$ kg m/m (35 g cm/cm) in tear strength.

Example 14

Using the refined hide dehydrated by centrifuging in the same manner as in Example 1, a 2.5% by weight dispersion of collagen in water of pH of 3.0 was prepared as in Example 13. The aqueous solution of de-N-acetylated chitin obtained in Example 1 and the aqueous solution of gelatin obtained in Example 2 were added to this dispersion to obtain an aqueous mixture of dispersed collagen fiber and gelatin and de-N-acetylated chitin at a weight ratio of 100:10:10. The thus prepared aqueous mixture was subjected to electrodeposition in the same manner as in Example 1 to prepare a tubular membrane, one of the composite materials of the present invention. The mechanical properties of the membrane, determined as in Example 1, were: $9\times10^{-6}$ m (9 μ) in thickness, $4.8\times10^{6}$ kg/m$^2$ (480 kg/cm$^2$) in tensile strength, 33% in elongation at break and $3.4\times10^{-2}$ kg m/m (34 g cm/cm) in tear strength.

Example 15

Into $2\times10^{-2}$ m$^3$ (20 litres) of Atkins-Pantin buffer solution of pH 9 containing 0.2% by weight of "Pronase" (manufactured by Kaken Chem. Co. Ltd., Japan), one kg of the refined hide obtained in Example 1 before the crosslinking treatment was immersed for 24 hours under slow stirring to bring the hide into reaction with Pronase. After the reaction was over, the lumpy material was recovered by centrifugation and washed with water to purity it. The purified material was dissolved into an aqueous 1N hydrochloric acid solution of pH of 3.5 to obtain 100 kg of an uniform solution of solubilized collagen. On determination of the concentration of solubilized collagen in the solution by taking and examining a small portion of the solution after freeze-drying, it was found to be 0.95% by weight.

To 13.5 kg of the aqueous 2.5% crosslinked collagen dispersion prepared in Example 1, 50 kg of de-ionized water were added under high speed stirring. 3.95 Kg of the thus obtained aqueous solution of solubilized collagen were added to this mixture. Then the whole mixture was stirred for 30 min. $7.48\times10^{-1}$

Kg (748 g) of the aqueous de-N-acetylated chitin solution prepared in Example 1 and $3.74\times10^{-1}$ kg (374 g) of the aqueous gelatin solution prepared in Example 2 were added to this mixture and the resulting mixture was stirred well. In the thus prepared aqueous mixture, the weight ratio of collagen fibers: soluble collagen: gelatin: de-N-acetylated chitin was 90:10:10:20.

By subjecting the aqueous mixture to electrodeposition while using the apparatus used in Example 1, a composite material, i.e. a tubular membrane, of the present invention was obtained of which the mechanical properties, determined as in Example 1, were: $9\times10^{-6}$ m (9 μ) in thickness, $5\times10^{6}$ kg/m$^2$ (500 kg/cm$^2$) in tensile strength, 35% in elongation at break and $3.5\times10^{-2}$ kg m/m (35 g cm/cm) in tear strength.

Example 16

$10^{-3}$ Kg (one gram) of acidic protease was added to $5\times10^{-2}$ kg (50 g) of an aqueous dispersion of 2.5% by weight of uncrosslinked collagen prepared following the procedures in Example 1. After keeping the mixture for 5 hours at 37°C, the mixture was neutralized and centrifuged. The solid matter recovered was washed with water, and then adjusted to pH 3.5 by the addition of an aqueous 1N hydrochloric acid solution. The thus prepared mixture, weighing 1 kg, was subjected to homogenization at a temperature lower than 10°C to finely divide it further. The thus obtianed liquid is hereinafter referred to as Liquid B.

A homogeneous solution of $2\times10^{-2}$ kg (20 g) of de-N-acetylated chitin dissolved in an aqueous 5% by weight of acetic acid solution, hereinafter referred to as Liquid A, was added to Liquid B, and the mixture was processed to form a homogeneous solution by a homogenizer.

Into a 3-litre flask provided with a stirrer, in which $2\times10^{-3}$ m$^3$ (2 litres) of decahydronaphthalene and $10^{-3}$ kg (one gram) of polyoxyethylene sorbitan (trade name Twin 80) were placed, $10^{-4}$ m$^3$ (100 ml) of the mixture of Liquid A and Liquid B was added. The whole mixture was stirred at 1,000 rpm for one hour to form a dispersion. By re-dispersing the dispersion in $10^{-2}$ m$^3$ (10 litres) of ethanol, insoluble matter was obtained. The insoluble matter was recovered by filtration and washed repeatedly with ethanol to remove the organic substances (except ethanol). The thus treated insoluble substance was re-dispersed in $10^{-3}$ m$^3$ (one litre) of an aqueous 2% by weight ammonium hydroxide solution. After neutralizing the dispersion, recovering the precipitate by filtration and washing with water, spherical particles of a composite material according to the present invention were obtained (Specimen H) of particle size of $10^{-4}$ to $10^{-3}$ m (0.1 to 1.0 mm) in diameter.

After freeze-drying Specimen H, $10^{-3}$ kg (one gram) of dried Specimen H was put into an aqueous physiological saline solution. The solution was filtered off to recover spherical particles. A glass column $8\times10^{-3}$ m (8 mm) in internal diameter was filled with these spherical particles. $5\times10^{-5}$ m$^3$ (50 ml) of rabbit's blood to which 250 units of heparin had been added were perfused through the column at a rate of $3.3\times10^{-7}$ m$^3$/s (20 ml/min) at 37°C for 15 min. After stopping the circulation, the blood was removed from the column as soon as possible. The column was washed with an aqueous physiological saline solution, and then the spherical particles were taken out from the column to examine the degree of adherence of platelets and blood corpuscles on the particles. The adherence of both the platelets and blood corpuscles was clearly noticeable.

Example 17

To $5\times10^{-2}$ kg (50 g) of the aqueous dispersion of crosslinked collagen fiber prepared in Example 1, $7.5\times10^{-3}$ kg (7.5 g) of the aqueous solution of gelatin of Example 2 were added. After adjusting the pH of the mixture by the addition of an 1N aqueous hydrochloric acid solution to 3.5, the mixture amounting to 1 kg was subjected at a temperature of lower than 10°C to a homogenizer to finely divide the collagen. The thus prepared dispersion is referred to as Liquid C.

A mixture of Liquid C and Liquid A of Example 16 was prepared and subjected to homogenization to obtain a homogeneous dispersion.

Into a 3-litre flask provided with a stirrer, in which $2\times10^{-3}$ m$^3$ (2 litres) of decahydronaphthalene and $10^{-3}$ kg (one gram) of polyoxyethylene sorbitan (Trade name Twin 80) were placed, $10^{-4}$ m$^3$ (100 ml) of the homogeneous dispersion of the mixture of Liquids C and A were introduced. After one hour of stirring at 1000 rpm, the mixture was re-dispersed into $10^{-2}$ m$^3$ (10 litres) of ethanol to obtain an insoluble material. The insoluble material was collected by filtration and washed repeatedly with ethanol to remove decahydronaphthalene. Then, the washed material was again dispersed in $10^{-3}$ m$^3$ (one litre) of an aqueous 2% by weight ammonium hydroxide solution. On neutralizing the dispersion, spherical particles were deposited. The particles were collected by filtration, and washed with water to obtain a composite material of the present invention (Specimen K). The spherical particles were $10^{-4}$ to $10^{-3}$ m (0.1 to 1.0 mm) in diameter. Results of examination of the surface of the particles of Specimen K as in Example 16 showed the adherence of platelets and blood corpuscles on the surface of the particles.

Example 18

After pouring the aqueous mixture of collagen fiber and de-N-acetylated chitin prepared in Example 6 onto a plate $2\times10^{-1}$ m (200 mm) square to a thickness of $5\times10^{-3}$ m (5 mm) and leaving for one hour, the plate and the mixture were immersed in an aqueous 0.1N ammonium hydroxide solution to neutralize the acidity, and washed with de-ionized water to remove salts. The thus obtained material was immersed in an aqueous 0.1M disodium phosphate solution containing 0.5% by weight of formaldehyde at 30°C for one

hour and then washed with water. The thus obtained material was freeze-dried. The material took the form of a sponge. The sponge showed an absorption of aqueous physiological saline solution of 20 kg per kg of the material. Further, it showed a strong coagulation activity to blood. Accordingly, it has been found that it is possible to use the material as a menstrual pad material and as a garrot tourniquet for urgent cases.

Example 19

The aqueous mixture of collagen fiber, gelatin and de-N-acetylated chitin of Example 2 was treated in the same manner as in Example 18 to obtain a sponge. This material showed an absorption ability to an aqueous physiological saline solution of 21 kg/kg material, and a strong coagulating property to human blood. Accordingly, it was found that the material is applicable for use in menstrual pads and tourniquets for urgent cases.

Example 20

$3\times10^{-2}$ kg (30 g) of the spherical particles (Specimen H of Example 16) were added to a solution prepared by dissolving $10^{-3}$ kg (1 g) of glucose-isomerase (2000 U/g; manufactured by Nagase Sangyo Co. Ltd.) in an aqueous 0.1 M disodium phosphate solution. After stirring the mixture for 2 hours at 5°C, $2\times10^{-6}$ $m^3$ (2 ml) of an aqueous 25% by weight glutaraldehyde solution was added to the mixture. After leaving the mixture for 5 hours, solid materials were recovered by filtration, washed with an aqueous phosphoric buffer solution of pH of 7.0 and added to $5\times10^{-4}$ $m^3$ (500 ml) of an aqueous phosphoric buffer solution containing $2\times10^{-3}$ kg (2 g) of glucose. After thus treating the mixture for 60 min at 70°C, the glucose and fructose contents of the solid material were $6\times10^{-4}$ kg and $1.4\times10^{-3}$ kg (600 and 1400 mg), respectively, and the amount of enzyme (glucose-isomerase) fixed to the material was 70% of the originally applied amount.

In addition, separately, the pH of the solution of collagen and de-N-acetylated chitin before re-dispersion in Example 16 was adjusted to 6.0. $10^{-3}$ Kg (1 g) of glucose-isomerase was added to the solution at 5°C. After re-dispersing the mixture as in Example 16, the solid matter was recovered by filtration and washed with ethanol and then with an aqueous phosphoric buffer solution of pH of 7.0. It was found that 85% of the original amount of enzyme (glucose-isomerase) was fixed onto the solid matter.

These results show that it is possible to use the material as a carrier for fixed enzymes and microbial bodies.

Example 21

The procedures of Example 20 were carried out except for using Specimen K obtained in Example 17 instead of Specimen H. The amounts of glucose and fructose on Specimen K after the treatment at 70°C for 60 min were $6\times10^{-4}$ and $1.4\times10^{-3}$ kg (600 and 1400 mg), respectively, and 70% of the original amount of the glucose-isomerase was fixed to the material.

From these results, it has been found that it is possible to employ a composite material according to the present invention, i.e. Specimen K, as a carrier of fixed enzymes and microbial bodies.

Example 22

After immersing Comparative Specimen A, comprising a shaped material consisting only of collagen fibers, in a homogeneous solution of a viscosity of $5\times10^{-2}$ Pas (50 cP) prepared by adding $5\times10^{-3}$ kg (5 g) of de-N-acetylated chitin having a degree of de-N-acetylation of 85% to $10^{-3}$ $m^3$ (one litre) of an aqueous 1% acetic acid solution at 20°C for one hour, the excess aqueous solution was removed. The thus treated material in a wet state was immersed once in an aqueous 0.1N ammonia solution. Then the shaped material was washed with water to remove any attached ammonium acetate and ammonia to obtain a composite material according to the present invention. This composite material showed the following mechanical properties: $3.95\times10^6$ kg/$m^2$ (395 kg/$cm^2$) in tensile strength, 33% of elongation at break and $5\times10^{-2}$ kg m/m (50 g cm/cm) of tear strength. The amount of de-N-acetylated chitin in the composite material relative to the collagen fibers was 2/100 by weight.

Example 23

In the manner of Example 22 except for using Comparative Specimen B obtained in Example 2 and a solution (viscosity: $10^{-1}$ Pas (100 cP)) of de-N-acetylated chitin having a degree of de-N-acetylation of 75%, a composite material of the present invention was obtained. The mechanical properties thereof were: $4\times10^6$ kg/$m^2$ (400 kg/$cm^2$) in tensile strength, 30% in elongation at break and $3\times10^{-2}$ kg m/m (30 g cm/cm) in tear strength. The content of de-N-acetylated chitin in the composite material obtained in this Example was 2 parts per 100 parts of collagen fiber.

**Claims**

1. A composite material comprising de-N-acetylated chitin and fibrous collagen in a weight ratio of de-N-acetylated chitin to fibrous collagen in the range 0.01:99.99 to 99:1.

2. A material according to claim 1, further comprising up to 40 parts by weight of gelatin and/or soluble collagen per 100 parts by weight of the composite material.

3. A material according to claim 1 or 2, wherein the fibrous collagen has been previously cross-linked or acylated.

4. A material according to any one of the preceding claims, which is further cross-linked.

5. A process for preparing a composite material as claimed in any one of the preceding claims, which process comprises so as to obtain the composite material, bringing de-N-acetylated chitin into contact with fibrous collagen and optionally gelatin and/or soluble collagen in an acidic medium and deacidifying the product thus obtained.

6. A process according to claim 5, wherein contact between the de-N-acetylated chitin and fibrous collagen is effected by mixing an aqueous acidic solution of the de-N-acetylated chitin and an aqueous dispersion of fibrous collagen or by immersing a material made of fibrous collagen in an aqueous acidic solution of the de-N-acetylated chitin.

7. A process according to claim 5 or 6, wherein the fibrous collagen has been cross-linked or acylated before contact with the de-N-acetylated chitin.

8. A process according to any one of claims 5 to 7, wherein deacidification is effected by the addition of an aqueous alkaline solution, by removal of the acid by evaporation or by electrodeposition.

9. A process according to any one of claims 5 to 8, wherein the resultant composite material is further cross-linked.

10. Use of a composite material as claimed in any one of claims 1 to 4 or which has been produced by a process as claimed in any one of claims 5 to 9 in food, or as a base material for immobilizing an enzyme.

11. A composite material as claimed in any one of claims 1 to 4 or which has been produced by a process as claimed in any one of claims 5 to 9 for use in medicine.

**Patentansprüche**

1. Verbundmaterial mit de-N-acetyliertem Chitin und faserigem Kollagen in einem Gewichtsverhältnis von de-N-acetyliertem Chitin zu faserigem Kollagen im Bereich von 0,01:99,99 bis 99:1.

2. Material nach Anspruch 1, welches ferner bis zu 40 Gew.- Teile Gelatine und/oder lösliches Kollagen pro 100 Gew.- Teile Verbundmaterial enthält.

3. Material nach Anspruch 1 oder 2, worin das fassrige Kollagen zuvor vernetzt oder acyliert wurde.

4. Material nach einem der voranstehenden Ansprüche, welches ferner vernetzt ist.

5. Verfahren zur Herstellung eines Verbundmaterials nach einem der voranstehenden Ansprüche, wobei zur Herstellung des Verbundmaterials das de-N-acetylierte Chitin in Kontakt mit faserigem Kollagen und gegebenenfalls mit Gelatine und/oder löslichem Kollagen in einem sauren Medium gebracht wird und das so erhaltene Produkt deacidifiziert wird.

6. Verfahren nach Anspruch 5, worin der Kontakt zwischen dem de-N-acetylierten Chitin und dem faserigen Kollagen bewirkt wird durch Vermischen einer wässrigen sauren Lösung des de-N-acetylierten Chitins und einer wässrigen Dispersion von faserigem Kollagen oder durch Eintauschen eines Materials aus faserigem Kollagen in eine wässrige saure Lösung des de-N-acetylierten Chitins.

7. Verfahren nach Anspruch 5 oder 6, worin das faserige Kollagen vor dem Kontakt mit dem de-N-acetylierten Chitin vernetzt oder acyliert wurde.

8. Verfahren nach einem der Ansprüche 5 bis 7, worin die Deacidifizierung durch Zugabe einer wässrigen alkalischen Lösung, durch Entfernung der Säure mittels Verdampfung oder durch Elektroabscheidung bewirkt wird.

9. Verfahren nach einem der Ansprüche 5 bis 8, worin das erhaltene Verbundmaterial weiter vernetzt wird.

10. Verwendung eines Verbundmaterials nach einem der Ansprüche 1 bis 4 oder welches nach einem der Verfahren gemäß einem der Ansprüche 5 bis 9 hergestellt wurde in Nahrungsmitteln oder als ein Basenmaterial zur Immobilisierung eines Enzyme.

11. Verbundmaterial nach einem der Ansprüche 1 bis 4 oder welches nach einem Verfahren gemäß einem der Ansprüche 5 bis 9 hergestellt wurde, zur Verwendung in der Medizin.

**Revendications**

1. Un matériau composite comprenant de la chitine dé-N-acétylée et du collagène fibreux dans un rapport pondéral chitine dé-N-acétylée/collagène fibreux compris dans l'intervalle de 0,1/99,99 à 99/1.

2. Un matériau selon la revendication 1, comprenant en outre jusqu'à 40 parties en poids de gélatine et/ou de collagène soluble pour 100 parties en poids du matériau composite.

3. Un matériau selon la revendication 1 ou 2, dans lequel le collagène fibreux a été au préalable réticulé ou acylé.

4. Un matériau selon l'une quelconque des revendications précédentes, qui est en outre réticulé.

5. Un procédé de préparation d'un matériau composite selon l'une quelconque des revendications précédentes, pour obtenir le matériau composite, consistant à mettre la chitine dé-N-acétylée au contact avec de collagène fibreux et éventuellement de gélatine et/ou de collagène soluble dans un milieu acide et à désacidifier le produit ainsi obtenu.

6. Un procédé selon la revendication 5, dans lequel le contact entre la chitine dé-N-acétylée et le

collagène fibreux s'effectue par mélange d'une solution acide aqueuse de chitine dé-N-acétylée et d'une dispersion aqueuse de collagène fibreux ou par immersion d'un matériau constitué de collagène fibreux dans une solution acide aqueuse de chitine dé-N-acétylée.

7. Un procédé selon la revendication 5 ou 6, dans lequel le collagène fibreux a été réticulé ou acylé par contact avec la chitine dé-N-acétylée.

8. Un procédé selon l'une quelconque des revendications 5 à 7, dans lequel la désacidification s'effectue par addition d'une solution alcaline aqueuse, par élimination de l'acide par évaporation ou par électrodéposition.

9. Un procédé selon l'une quelconque des revendications 5 à 8, dans lequel le matériau composite résultant est en outre réticulé.

10. Utilisation d'un matériau composite selon l'une quelconque des revendications 1 à 4, ou produit par un procédé selon l'une quelconque des revendications 5 à 9, dans des produits alimentaires ou comme matériau de base pour immobiliser une enzyme.

11. Un matériau composite selon l'une quelconque des revendications 1 à 4, qui a été produit par un procédé tel que revendiqué dans l'une quelconque des revendications 5 à 9, pour utilisation médicale.